# EUROPEAN PATENT APPLICATION

(11) **EP 4 787 178 A1**
(43) Date of publication of application: **05.08.2026**
(21) Application number: 24869767.4
(22) Date of filing: 23.05.2024
(51) Int. Cl.: G06F 13/42

(54) **ELECTROENCEPHALOGRAPHY SIGNAL ACQUISITION AND PROCESSING DEVICE AND METHOD**

(30) Priority: 26.09.2023 CN 202311255915
(71) Applicant: Kingfar International Inc., Beijing 100085 (CN); Nanjing Kingfar Health Technology Inc., Nanjing, Jiangsu 211112 (CN)
(72) Inventor: ZHAO, Qichao, Beijing 100085 (CN); YANG, Ran, Beijing 100085 (CN)
(74) Representative: Meyer-Dulheuer MD Legal Patentanwälte PartG mbB
(86) International application number: PCT/CN2024/094831
(87) International publication number: WO 2025/066201

(57) **Abstract**

Provided are a device and method for acquiring and processing an electroencephalogram signal. The device includes an analog front-end processing circuit including an analog front-end chip, a frequency division circuit connected to an external clock with a first clock frequency, and a control unit. The analog front-end chip is configured to take a clock signal with the first clock frequency from an external clock as a clock input, acquire an EEG signal to obtain an analog EEG signal, convert the analog EEG signal into a digital EEG signal, and transmit the digital EEG signal to the control unit. The frequency division circuit is configured to perform N-fold frequency division on a clock crystal oscillator of the external clock to obtain a clock signal with a target clock frequency, where N is an even multiple of 2 and N=f1/fg, where f1 represents the first clock frequency, and fg represents the target clock frequency. The control unit is configured to take the clock signal with the target clock frequency obtained after frequency division by the frequency division circuit as a clock input, and perform data processing on the digital EEG signal from the analog front-end chip.

## Description

The present application claims priority to Chinese Patent Application No. 202311255915.2, September 26, 2023, and titled "Device and Method for Acquiring and Processing Electroencephalography Signal", the content of which is incorporated herein by reference in its entirety.

### TECHNICAL FIELD

The present disclosure relates to the field of signal processing technologies, and in particular, to a device and a method for acquiring and processing an electroencephalography signal.

### BACKGROUND

The main neuroelectrophysiological signals include electroencephalography (EEG) signals acquired on the scalp surface, electromyography (EMG) signals, local field potentials acquired in the brain, and spike signals directly corresponding to neural activities. These signals all have different characteristics, such as different amplitudes and frequency bands, and the electrodes acquiring them also have many different properties, such as different impedances, and different electrode polarization voltages also form different offsets.

At present, the development of high-precision analog-to-digital converters (ADCs) enables the acquisition of these neurophysiological signals generally implemented by analog front-end chips. The recommended external clock frequency for many high-precision analog front-end (or analog integrated front-end) chips is 2.048Mhz, and the corresponding sampling rate at this time is 250Hz.

In the process of implementing the present disclosure, the applicant has found that some applications require the synchronous acquisition (for example, multi-channel synchronous acquisition) and processing of multiple physiological signals. For example, it is necessary not only to acquire EEG signals, but also to acquire physiological signals such as electrodermal activity (EDA) signals, photo plethysmo graphy (PPG) signals, eye movement signals, heart rate signals, and/or functional near-infrared spectroscopy (fNIRS) signals. The acquisition of these physiological signals usually adopts sampling rates of 8Hz, 16Hz, 64Hz, 128Hz or 256Hz, etc. Therefore, the processing of these signals needs to adopt a data processing unit (main control chip) with a sampling rate of 256Hz or an integer multiple of 256Hz. However, the main control chip MCU with a sampling rate of 256Hz or an integer multiple of 256Hz cannot be used in conjunction with the analog front-end chip with a sampling rate of 250Hz. This is because the sampling rate required by the main control chip MCU is 256Hz or an integer multiple of 256Hz, and when the analog front-end chip with a sampling rate of 250 Hz is used in conjunction with the selected main control chip for acquisition, only the sampling rate of the EEG signal of 250Hz or an integer multiple of 250 Hz can be achieved, making it difficult for the main control chip to perform data processing. If the sampling rate of 256Hz is forcibly used for acquisition, there will be a phenomenon of clock mismatch between the analog front-end chip and the main control unit, which will lead to abnormal acquisition of physiological signal data. Therefore, EEG signals and other physiological signals cannot be processed synchronously by the same main control chip clock.

Therefore, how to solve the clock asynchronization and collaborative processing between data acquisition and data processing is an urgent problem to be solved.

### SUMMARY

In view of this, the present disclosure provides a device for acquiring and processing an electroencephalogram signal and a frequency division circuit, to implement cooperative acquisition and time synchronization between an analog front-end chip and a main control chip with a sampling rate of 256Hz.

One aspect of the present disclosure provides a device for acquiring and processing an electroencephalogram signal. The device includes an analog front-end processing circuit, a frequency division circuit, and a control unit. The analog front-end processing circuit includes an analog front-end chip. The analog front-end chip is configured to: take a clock signal with a first clock frequency from an external clock as a clock input, acquire an electroencephalography (EEG) signal at a first sampling rate matching the first clock frequency to obtain an analog EEG signal, convert the analog EEG signal into a digital EEG signal, and transmit the digital EEG signal to the control unit. A default sampling rate of the analog front-end chip is a second sampling rate different from the first sampling rate. The frequency division circuit is connected to the external clock with the first clock frequency and configured to perform N-fold frequency division on a clock crystal oscillator of the external clock to obtain a clock signal with a target clock frequency, where N is an even multiple of 2 and N=f1/fg, where f1 represents the first clock frequency, and fg represents the target clock frequency. The control unit is configured to take the clock signal with the target clock frequency obtained after frequency division by the frequency division circuit as a clock input, to perform data processing on the digital EEG signal from the analog front-end chip.

In some embodiments of the present disclosure, the device further includes a power supply circuit. The power supply circuit is configured to supply power for the analog front-end chip and the main control chip. The power supply circuit includes a main power supply module and an auxiliary power supply module. The main power supply module includes an analog circuit power supply circuit and a digital circuit power supply circuit. The analog circuit power supply circuit includes a low dropout regulator, and is configured to provide a first voltage for an analog circuit of the EEG signal acquiring device to supply power. The digital circuit power supply circuit includes a buck circuit, and is configured to provide a first voltage for a digital circuit of the electroencephalogram signal acquiring device to supply power. The auxiliary circuit module is configured to divide a second voltage from a voltage provided by the analog circuit power supply circuit to supply power to the analog front-end chip.

In some embodiments of the present disclosure, the first clock frequency ranges from 1.5MHz to 2.25MHz, and the first sampling rate is F and satisfies: F=f1/2/k, where k is selected from 64, 128, 256, 512, 1024, 2048, or 4096.

In some embodiments of the present disclosure, the first clock frequency is 2.097MHz, N is 64, the first sampling rate is 256Hz or a multiple of 256Hz, and the target clock frequency is 32.768KHz.

In some embodiments of the present disclosure, the frequency division circuit includes a first frequency division sub-circuit and a second frequency division sub-circuit. The first frequency division sub-circuit is connected to the external clock with the first clock frequency, and configured to perform M-fold frequency division on the clock crystal oscillator of the external clock, where M is an even multiple of 2. The second frequency division sub-circuit is configured to further perform L-fold frequency division on a signal after the M-fold frequency division by the first frequency division circuit to obtain the clock signal with the target clock frequency, where L is an even multiple of 2 and L=N/M.

In some embodiments of the present disclosure, the frequency division circuit includes a first frequency division sub-circuit, a second frequency division sub-circuit and a third frequency division sub-circuit. The first frequency division sub-circuit is connected to the external clock of the first clock frequency, and configured to perform 4-fold frequency division on the clock crystal oscillator of the external clock. The second frequency division sub-circuit is configured to further perform 4-fold frequency division on a signal after the 4-fold frequency division by the first frequency division circuit. The third frequency division sub-circuit is configured to further perform 4-fold frequency division on a signal after the 4-fold frequency division by the second frequency division circuit to obtain the clock signal with 32.768KHz.

In some embodiments of the present disclosure, the analog front-end processing circuit further includes an electrostatic discharge (ESD) protection circuit. The electrostatic discharge (ESD) protection circuit is disposed at an EEG signal input end for ESD protection.

In some embodiments of the present disclosure, the analog front-end processing circuit further includes a low-pass filtering circuit. The low-pass filtering circuit is disposed at the EEG signal input end for filtering out high-frequency electromagnetic waves and high-frequency crosstalk.

In some embodiments of the present disclosure, the analog front-end chip includes a multi-channel EEG signal multiplexer (MUX) switching module configured to perform polling acquisition on the EEG signal.

In some embodiments of the present disclosure, the first voltage ranges from 1.8V to 3.6V, preferably 3.0V. The second voltage is represented as AVDD and satisfies: AVDD=(AVDD+)-(AVDD-), where AVDD+ and AVDD- represent a high voltage and a low voltage of the analog front-end chip respectively, and AVDD ranges from 4.75V to 5.25V.

In another aspect of the present disclosure, a method for acquiring and processing an EEG signal is further provided, which includes: taking, by an analog front-end chip, a clock signal with a first clock frequency from an external clock as a clock input, acquiring an EEG signal at a first sampling rate matching the first clock frequency to obtain an analog EEG signal, converting the analog EEG signal into a digital EEG signal, and transmitting the digital EEG signal to a control unit, where a default sampling rate of the analog front-end chip is a second sampling rate different from the first sampling rate; connecting, by a frequency division circuit, the external clock with the first clock frequency, and performing N-fold frequency division on a clock crystal oscillator of the external clock to obtain a clock signal with a target clock frequency, where N is an even multiple of 2 and N=f1/fg, where f1 represents the first clock frequency, and fg represents the target clock frequency; and taking, by the control unit, the clock signal with the target clock frequency obtained after frequency division by the frequency division circuit as a clock input, and performing data processing on the digital EEG signal from the analog front-end chip.

In some embodiments of the present disclosure, the connecting, by a frequency division circuit, the external clock with the first clock frequency, and performing N-fold frequency division on a clock crystal oscillator of the external clock to obtain a clock signal with a target clock frequency includes: connecting, by the frequency division circuit, the external clock with the first clock frequency, and performing M-fold frequency division on the clock crystal oscillator of the external clock, where M is an even multiple of 2; and performing L-fold frequency division on a signal after the M-fold frequency division by the first frequency division circuit to obtain the clock signal with the target clock frequency, where L is an even multiple of 2 and L=N/M.

In some embodiments of the present disclosure, the connecting, by a frequency division circuit, the external clock with the first clock frequency, and performing N-fold frequency division on a clock crystal oscillator of the external clock to obtain a clock signal with a target clock frequency includes: connecting, by the frequency division circuit to be connected to the external clock of the first clock frequency; performing a first 4-fold frequency division on the clock crystal oscillator of the external clock; performing a second 4-fold frequency division on a signal after the first 4-fold frequency division; and performing a third 4-fold frequency division on a signal after the second 4-fold frequency division to obtain the clock signal with the target clock frequency.

The device and method for acquiring and processing an EEG signal can realize the cooperative acquisition between the analog front-end chip and the main control chip, enabling synchronous process of multiple physiological signals including EEG signals by the same main control chip with a selected sampling rate (such as 256Hz), which greatly expands the application scenarios of physiological signals.

Additional advantages, objects, and features of the present disclosure will be set forth partially in the following description, and will be partially apparent to those of ordinary skill in the art after studying the following content, or can be learned through the practice of the present disclosure. The objects and other advantages of the present disclosure can be achieved and obtained by the structures specifically pointed out in the specification and accompanying drawings.

Those skilled in the art will appreciate that the objects and advantages that can be achieved with the present disclosure are not limited to the above detailed description, and the above and other objects that can be achieved by the present disclosure will be more clearly understood according to the following detailed description.

### BRIEF DESCRIPTION OF DRAWINGS

The accompanying drawings described herein are used to provide a further understanding of the present disclosure, constitute a part of the present disclosure, and do not limit the present disclosure.
FIG. 1 is a schematic diagram of a device for acquiring and processing an EEG signal according to an embodiment of the present disclosure.

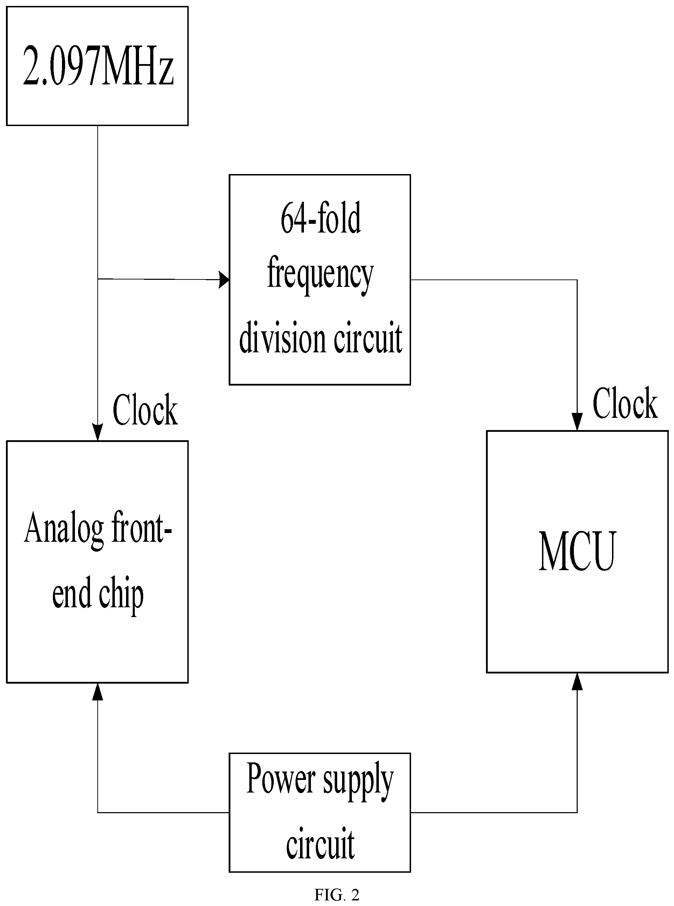

FIG. 2 is another schematic diagram of a device for acquiring and processing an EEG signal according to an embodiment of the present disclosure.
FIG. 3 is a schematic diagram of an external clock circuit.
FIG. 4 is a schematic diagram of a frequency division circuit according to an embodiment of the present disclosure.
FIG. 5 is a schematic flowchart of a method for acquiring and processing an EEG signal according to an embodiment of the present disclosure.
FIG. 6 is a structural schematic diagram of an analog front-end processing circuit according to an embodiment of the present disclosure.

### DESCRIPTION OF EMBODIMENTS

In order to make the objectives, technical solutions, and advantages of the present disclosure clearer, the following further describes the present disclosure in detail below with reference to the embodiments and accompanying drawings. Herein, the exemplary embodiments and descriptions of the present disclosure are used to explain the present disclosure, but are not intended to limit the present disclosure.

Here, it should also be noted that, in order to avoid obscuring the present disclosure due to unnecessary details, only structures and/or processing steps closely related to the solution according to the present disclosure are shown in the accompanying drawings, and other details not related to the present disclosure are omitted.

It should be emphasized that the term "comprise/include" used herein refers to the existence of features, elements, steps, or components, but does not exclude the existence or addition of one or more other features, elements, steps, or components.

As mentioned above, when the analog front-end chip with a sampling rate of 250Hz is used in conjunction with the main control chip (MCU) with a sampling rate of 256Hz for acquisition, only the EEG signal sampling rate of 250Hz or an integer multiple of 250Hz can be achieved, making it difficult for the main control chip to perform data processing. If the analog front-end chip forcibly uses a sampling rate of 256Hz for acquisition, there may be a phenomenon of clock mismatch between the analog front-end chip and the main control unit, which will lead to abnormal acquisition of physiological signal data. Therefore, EEG signals and other physiological signals cannot be processed synchronously by the same main control chip clock.

In addition, for an analog front-end chip whose default or fixed sampling rate is neither 250Hz or a multiple thereof, nor 256Hz or a multiple thereof, the same problem also exists when the sampling rate of 256Hz is used for sampling.

To solve this technical problem, the present disclosure provides an improved device for acquiring and processing an EEG signal, which converts a fixed sampling rate of 250Hz (or other sampling rates different from 256Hz and multiples thereof) of an analog integrated front-end into a custom sampling rate of 256Hz by changing an external input clock, which can realize the cooperative acquisition between the analog front-end chip and the main control chip, thereby realizing synchronous process of multiple physiological signals including EEG signals by the main control chip with a sampling rate of 256Hz, using the multiple physiological signals to collaboratively determine a tested physiological state, and thus greatly expanding the application scenarios of physiological signals.

The analog front-end chip supports input of an external clock. Taking an analog front-end chip with a sampling rate of 250Hz as an example, the allowable input range of the external clock is 1.5 MHz to 2.25MHz, and the recommended external clock input is 2.048MHz (the sampling rate of 250Hz is obtained based on (2.048MHz/2/4096)). Within the allowable range, the present disclosure does not adopt the recommended external clock input of 2.048MHz, but selects a clock of 2.097MHz. The clock of 2.097MHz is an integer multiple of 32.768KHz, and 32.768KHz is exactly a clock of the main control chip, thereby modifying the external input clock of the analog front-end chip to 2.097 MHz, and converting an analog integrated front-end with a fixed sampling rate of 250Hz into a sampling rate of 256Hz. However, while changing the external input clock to 2.097MHz to realize the sampling of the analog integrated front-end at a sampling rate of 256Hz, a defect is caused, that is, there may be a problem of clock asynchronization between the analog integrated front-end and the main control chip due to frequency offset, that is, a clock deviation may also occur between the analog integrated front-end and the main control chip due to the clock asynchronization. In this regard, further, in order to eliminate the clock deviation and ensure the clock synchronization between the analog integrated front-end and the main control chip, not only 2.097MHz is used as the clock input of the analog integrated front-end, but also a frequency division circuit is designed to perform 64-fold frequency division on 2.097 MHz to obtain 32.768kHz, which is used as the clock input of the main control chip to replace the original 32.768kHz clock of the main control chip, thereby realizing the unification of clock source, and thus realizing the clock synchronization. In this way, the purpose of clock synchronization between the analog integration front-end and the main control chip can be achieved while the analog integration front-end adopts the sampling rate of 256Hz for EEG signal acquisition.

For another analog front-end chip whose default or fixed sampling rate is neither 250Hz or a multiple thereof, or 256Hz or a multiple thereof, the present disclosure also needs to switch to a custom sampling rate (for example, 256Hz) for sampling, and a clock frequency of an external clock of the analog front-end chip is changed from a fixed or default clock frequency to a clock frequency matching the target sampling rate, so that EEG signal acquisition can be performed according to a first sampling rate matching an externally input clock frequency to obtain the EEG analog signal. Correspondingly, in order to realize clock synchronization between the analog integrated front-end and the main control chip, the frequency division circuit is used to perform frequency division on the externally input clock frequency to obtain a target clock frequency consistent with that of the main control chip, and then input it to the main control chip.

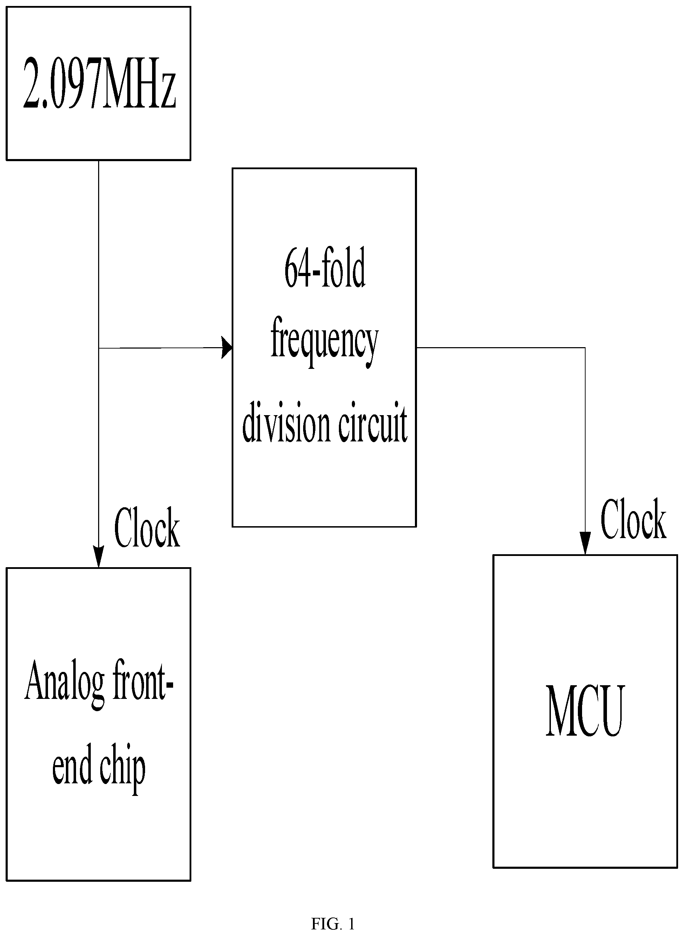

FIG. 1 is a structural schematic diagram of a device for acquiring and processing an EEG signal according to an embodiment of the present disclosure. As shown in FIG. 1, the device for acquiring and processing an EEG signal includes an analog front-end processing circuit, a frequency division circuit and a main control chip (MCU).

The analog front-end processing circuit includes an analog front-end chip. The analog front-end chip is configured to take a clock signal with a first clock frequency from an external clock as a clock input, acquire an EEG signal at a first sampling rate matching the first clock frequency to obtain the analog EEG signal, convert the analog EEG signal into a digital EEG signal, and transmit the digital EEG signal to a control unit. A default sampling rate of the analog front-end chip is a second sampling rate different from the first sampling rate.

In an embodiment of the present disclosure, the first clock frequency f1 ranges from 1.5MHz to 2.25MHz, and the first sampling rate F matching the first clock frequency satisfies: F=f1/2/k, where k is selected from 64, 128, 256, 512, 1024, 2048, or 4096. As an example, the first clock frequency is 2.097MHz, the first sampling rate is 256Hz or a multiple of 256Hz, and the second sampling rate is 250Hz or a multiple of 250Hz. Then the analog front-end chip is configured to take a clock signal with 2.097 MHz from an external clock as a clock input, acquire an EEG signal to obtain an analog EEG signal, convert the analog EEG signal into a digital EEG signal, and transmit the digital EEG signal to a control unit (i.e., the main control chip MCU).

The frequency division circuit is connected to an external clock with the first clock frequency and configured to perform N-fold frequency division on a clock crystal oscillator of the external clock to obtain a clock signal with a target clock frequency, where N is an even multiple of 2 and N=f1/fg, where f1 represents the first clock frequency, and fg represents the target clock frequency.

When the first clock frequency is 2.097MHz and the first sampling rate is 256Hz or a multiple of 256Hz, the target clock frequency is 32.768KHz, and N is 64. Then the frequency division circuit is connected to an external clock of 2.097MHz and configured to perform 64-fold frequency division on the clock crystal oscillator of 2.097MHz to obtain a clock signal of 32.768 kHz.

The main control chip MCU is configured to take the clock signal with the target clock frequency obtained after frequency division by the frequency division circuit as a clock input, and perform data processing on the digital EEG signal from the analog front-end chip.

In some other embodiments of the present disclosure, the device for acquiring and processing the EEG signal further includes a power supply circuit, configured to supply power to the analog front-end chip and the main control chip.

The power supply circuit can provide digital circuit power supply of the first voltage, analog circuit power supply of the first voltage and the second voltage, which are used to respectively provide the required voltages to the digital circuits and analog circuits in the devices including the analog front-end chip and the main control chip in the device for acquiring and processing the EEG signal. The first voltage ranges from 1.8V to 3.6V, preferably 3.0V. The second voltage is represented as AVDD and satisfies: AVDD=AVDD+-AVDD-, where AVDD+ and AVDD- represent a high voltage and a low voltage of the analog front-end chip respectively, and AVDD ranges from 4.75V to 5.25V, preferably 5V. As an example, the power supply circuit may include a main power supply module and an auxiliary supply module. The main power supply module includes a power supply management chip. The power supply management chip includes an analog circuit power supply circuit and a digital circuit power supply circuit. The analog circuit power supply circuit includes a low dropout regulator (LDO) for supplying a power supply voltage of 3.0V to the analog circuit of the EEG signal acquiring device. The digital circuit power supply circuit includes a buck circuit (BUCK circuit) for supplying a power supply voltage of 3.0V to the digital circuit of the EEG signal acquiring device.

The auxiliary circuit module can divide ±2.5V from the voltage provided by the analog circuit power supply circuit to supply a power supply voltage of ±2.5V to the analog front-end chip, that is, AVDD+ is +2.5V and AVDD- is -2.5V. For example, a provided analog circuit supply voltage of 3.0V is first boosted to output 5V, and the 5V is output a power supply voltage of 2.5V through a buck chip. 5V is converted to -5V, and then -5V is converted to -2.5V, so that a power supply voltage of -2.5V is obtained.

Since the power supply circuit can be implemented by using an existing circuit capable of realizing digital circuit power supply of 3.0V and analog circuit power supply of 3.0 V and ±2.5V, and details are not described herein again.

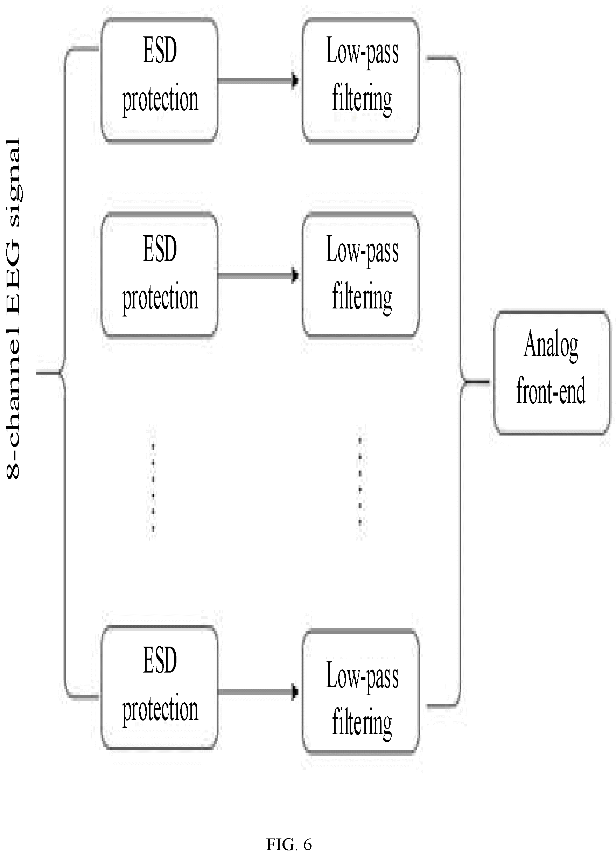

In some embodiments of the present disclosure, as shown in FIG. 6, in addition to the analog front-end chip, the analog front-end processing circuit may further include an electrostatic protection (ESD) circuit. The ESD circuit is disposed at the EEG signal input end for ESD of each acquisition channel. When receiving lightning surge and ESD electrostatic discharge or other instantaneous voltage, the device can perform a protection effect on the circuit, thereby preventing the device from being damaged by transient lightning surge and ESD electrostatic.

In some embodiments of the present disclosure, as shown in FIG. 6, the analog front-end processing circuit may further include a low-pass filtering circuit. The low-pass filtering circuit is disposed at the EEG signal input end and is configured to: filter out high-frequency electromagnetic waves for each acquisition channel, and filter out high-frequency crosstalk for each acquisition channel.

When the conducting current exists on the wire, the higher the frequency of the current is, the easier it is to form radiation, thereby generating strong radiation interference. Therefore, filtering out these high-frequency noises may effectively reduce cable radiation.

Since distributed capacitance and mutual inductance exist between wires or cables, mutual crosstalk can be generated. These interferences are mainly high-frequency, so low-pass filtering can remove these interferences.

In some embodiments of the present disclosure, the interior of the analog front-end chip includes: a multi-channel EEG signal MUX switching module (such as 8-channel or 16-channel, etc., the present disclosure is not limited thereto), a programmable gain module, an analog-to-digital signal conversion module, a right leg driving module (right leg driving circuit), and the like.

The EEG signal measured by the analog front-end chip may be an 8-channel EEG signal or a 16-channel EEG signal. After the 8-channel EEG signal or the 16-channel EEG signal is input the analog front-end chip, data acquisition is not performed simultaneously, but polling data acquisition is performed through the MUX switching module. The programmable gain module can amplify the EEG signal by 1x, 2x, 4x, 6x, 8x, 12x, or 24x, etc. The amplified analog signal is converted into a digital signal through the analog-to-digital signal conversion module, and then transmit the digital signal to the MCU by communicating with the MCU through the Serial Peripheral Interface (SPI) protocol. In addition, the right leg driving module is configured to reduce common mode interference. After amplifying the EEG signal, the common mode component is output by the programmable gain module, then sent to the right leg driving module, and then returned to the human body, thereby reducing interference. Since the multi-channel EEG signal MUX switching module, the programmable gain module, the analog-to-digital signal conversion module and the right leg driving module are modules in the existing analog front-end chip, details are not described herein again.

In some embodiments of the present disclosure, the frequency division circuit may include 2, 3, or more frequency division sub-circuits. For example, the frequency division circuit may include a first frequency division sub-circuit and a second frequency division sub-circuit.

The first frequency division sub-circuit is connected to the external clock with the first clock frequency, and configured to perform M-fold frequency division on the clock crystal oscillator of the external clock, where M is an even multiple of 2.

The second frequency division sub-circuit is configured to further perform L-fold frequency division on a signal after the M-fold frequency division by the first frequency division circuit to obtain a clock signal with a target clock frequency, where L is an even multiple of 2 and L=N/M.

When N=64, if the 64-fold frequency division circuit includes 2 frequency division sub-circuits, the 64-fold frequency division circuit may include a first frequency division sub-circuit and a second frequency division sub-circuit.

The first frequency division sub-circuit is connected to an external clock of 2.097MHz, and configured to perform M-fold frequency division on a clock crystal oscillator of 2.097MHz, where M is an even multiple of 2, preferably M is 4, 8, or 16.

The second frequency division sub-circuit is configured to further perform L-fold frequency division on a signal after the M-fold frequency division by the first frequency division circuit to obtain a clock signal of 32.768kHz, where L=64/M.

That is, the crystal oscillator of 2.097Mhz may be subjected to 16-fold frequency division, then further subjected to 4-fold frequency division, so as to realize 64-fold frequency division in total, and finally 32.768kHz is output as an input clock of the MCU. The input clock is an input clock from a same source as the analog integrated front-end. Alternatively, the crystal oscillator of 2.097Mhz may be subjected to 8-fold frequency division, then further subjected to 8-fold frequency division, so as to realize 64-fold frequency division in total, and finally 32.768kHz is output as an input clock from a same source as the analog integrated front-end. Alternatively, the crystal oscillator of 2.097Mhz may be subjected to 4-fold frequency division, then further subjected to 16-fold frequency division, so as to realize 64-fold frequency division in total, and finally 32.768kHz is output as an input clock from a same source as the analog integrated front-end.

When the 64-fold frequency division circuit includes 3 frequency division sub-circuits, the 64-fold frequency division circuit may include a first frequency division sub-circuit, a second frequency division sub-circuit and a third frequency division sub-circuit.

The first frequency division sub-circuit is connected to an external clock of 2.097MHz, and configured to perform 4-fold frequency division on a clock crystal oscillator of 2.097MHz.

The second frequency division sub-circuit is configured to further perform 4-fold frequency division on a signal after the 4-fold frequency division by the first frequency division circuit.

The third frequency division sub-circuit is configured to further perform 4-fold frequency division on a signal after the 4-fold frequency division by the second frequency division circuit to obtain a clock signal with a target clock frequency.

That is, the first frequency division sub-circuit, the second frequency division sub-circuit and the third frequency division sub-circuit are same 4-fold frequency division circuits.

In some other embodiments of the present disclosure, more frequency division sub-circuits may also be included, with a frequency division multiple of 2-fold to 16-fold, which will not be listed one by one here.

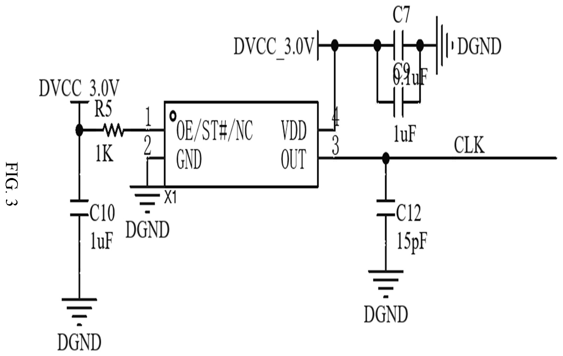

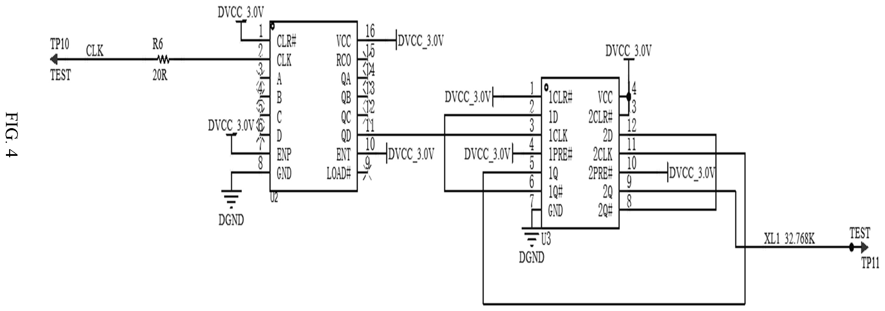

FIG. 3 is a schematic diagram of an external clock circuit according to an embodiment of the present disclosure. FIG. 4 is a schematic diagram of a frequency division circuit according to an embodiment of the present disclosure. As shown in FIG. 3, the pin 3 of the clock circuit X1 outputs a clock of 2.097MHz, which is output to the analog front-end chip as a clock source of the analog front-end chip on one hand, and to the pin 2 of the first frequency division sub-circuit (chip U6, which is a 16-fold frequency division circuit) as a main input of the frequency division circuit on the other hand. Then, after being subjected to 16-fold frequency division by chip U6, the clock is output from the pin 11, and then input from the pin 3 of the second frequency division sub-circuit (chip U8, which is a 4-fold frequency division circuit). After being subjected to 4-fold frequency division by chip U8, the clock of 32.768KHz is output from the pin 11 and sent to the main control chip MCU as a clock signal of the MUC, thereby not only realizing that the analog front-end chip can acquire EEG signals at 256Hz, but also realizing clock synchronization between the analog front-end chip and the main control chip.

In some embodiments of the present disclosure, the main control chip MCU is a chip integrating Bluetooth and wired communication. After the ADC of the analog front-end chip transmits data (digital signals) to the MCU through the SPI protocol, the MCU processes the data and further transmits the processed data to the host computer through the Bluetooth protocol or wired transmission manner.

The MCU mainly includes an indicator light circuit control module, an analog integrated front-end EEG signal data receiving and processing module, a power circuit control module, a Lead-off switching control (disconnection detection control) module, an EEG signal switching control module, and a two-channel biological (BIO) signal receiving and processing module.

The indicator light circuit control module is configured to control a charging indicator light and a Bluetooth indicator light.

The analog integrated front-end EEG signal data receiving and processing module is configured to receive and process the EEG digital signal from the analog integrated front-end.

The power circuit control module is configured to perform power monitoring and charging detection, and perform corresponding charging control.

The Lead-off switching control (disconnection detection control) module is configured to perform polling detection of the connection degree of multi-channel impedance.

The EEG signal switching control module is configured to perform polling detection on multi-channel EEG signals.

The two-channel BIO signal receiving and processing module is used for physiological signal detection.

The main control chip MCU may be implemented by using an existing MCU chip, or may be a self-studied chip capable of implementing a similar function.

It can be seen from the above that the present disclosure can convert the analog integrated front-end with a fixed sampling rate of 250Hz into one with a sampling rate of 256Hz by means of hardware, and solve the problem of clock asynchronization caused by changing the sampling rate through the frequency division circuit, thereby realizing the synchronization processing of multiple physiological signals including EEG signals by a same main control chip with a sampling rate of 256Hz, and thus greatly expanding the application scenarios of physiological signals.

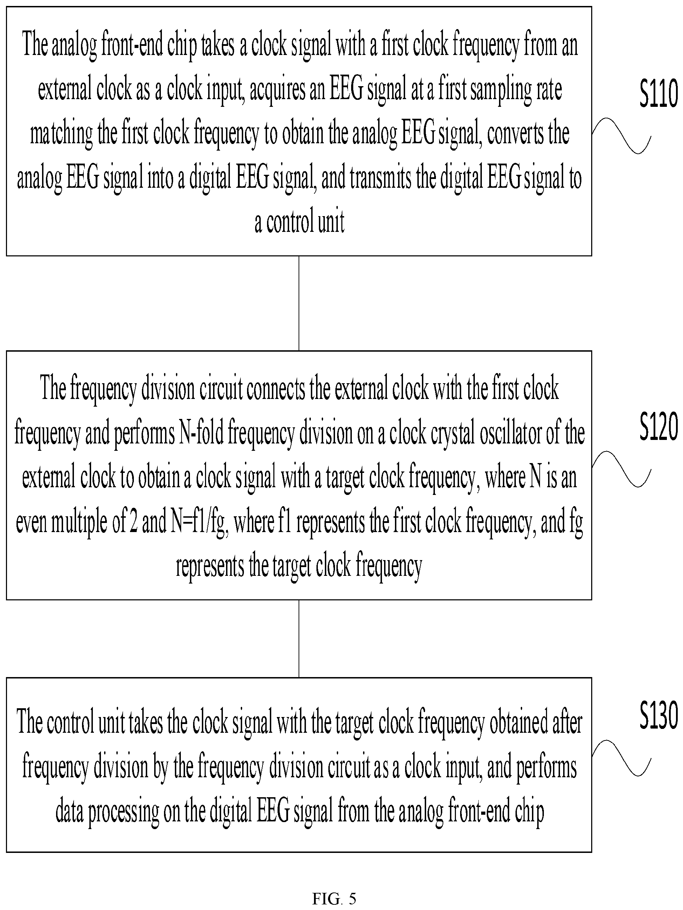

Corresponding to the foregoing device, the present disclosure further provides a method for acquiring and processing an EEG signal, and as shown in FIG. 5, the method includes the following steps of S110, S120, and S130.

In step S110, the analog front-end chip takes a clock signal with a first clock frequency from an external clock as a clock input, acquires an EEG signal at a first sampling rate matching the first clock frequency to obtain the analog EEG signal, converts the analog EEG signal into a digital EEG signal, and transmits the digital EEG signal to a control unit. A default sampling rate of the analog front-end chip is a second sampling rate different from the first sampling rate.

In the previous example, the first clock frequency is 2.097MHz, the first sampling rate is 256Hz or a multiple of 256Hz, and the second sampling rate is 250Hz.

In step S120, the frequency division circuit connects the external clock with the first clock frequency and performs N-fold frequency division on a clock crystal oscillator of the external clock to obtain a clock signal with a target clock frequency, where N is an even multiple of 2 and N=f1/fg, where f1 represents the first clock frequency, and fg represents the target clock frequency.

In the previous example, the target clock frequency is 32.768KHz and N is 64.

In step S130, the control unit takes the clock signal with the target clock frequency obtained after frequency division by the frequency division circuit as a clock input, and performs data processing on the digital EEG signal from the analog front-end chip.

As an example, the frequency division circuit is connected to the external clock with the first clock frequency and performs N-fold frequency division on a clock crystal oscillator of the external clock to obtain a clock signal with a target clock frequency, which includes that: the frequency division circuit is connected to the external clock of the first clock frequency, and performs M-fold frequency division on the clock crystal oscillator of the external clock, where M is an even multiple of 2; and further performs L-fold frequency division on a signal after the M-fold frequency division by the first frequency division circuit to obtain a clock signal of 32.768KHz, where L is an even multiple of 2 and L=N/M.

As another example, when the first clock frequency is 2.097MHz, N is 64, the first sampling rate is 256Hz or a multiple of 256Hz, and the target clock frequency is 32.768KHz, the frequency division circuit is connected to the external clock with the first clock frequency and performs N-fold frequency division on a clock crystal oscillator of the external clock to obtain a clock signal with a target clock frequency, which includes that: the frequency division circuit is connected to the external clock of 2.097MHz, and performs a first 4-fold frequency division on the clock crystal oscillator of 2.097MHz; further performing a second 4-fold frequency division on a signal after the first 4-fold frequency division; and further performing a third 4-fold frequency division on a signal after the second 4-fold frequency division to obtain a clock signal of 32.768KHz.

It should be understood by those of ordinary skill in the art that the components, systems, and methods described in conjunction with the embodiments disclosed herein can be implemented in hardware, software, or a combination of the two. Whether these functions are executed in hardware or software depends on the specific application and design constraints of the technical solution. Those skilled in the art may use different methods to implement the described functions for each specific application, but such implementation should not be considered to be beyond a scope of the present disclosure. When implemented in hardware, it may be, for example, an electronic circuit, an application specific integrated circuit (ASIC), appropriate firmware, a plug-in, a function card, or the like. When implemented in software, elements of the present disclosure are programs or code segments used to perform a required task. The program or code segments may be stored in a machine-readable medium or transmitted over data signals carried in a carrier wave on a transmission medium or communication link.

It should be clear that the present disclosure is not limited to the specific configurations and processes described above and illustrated in the drawings. Detailed descriptions of known methods are omitted here for the sake of brevity. In the above embodiments, many specific steps are described and illustrated as examples. However, the method process of the present disclosure is not limited to the specific steps described and shown, and those skilled in the art may make various changes, modifications and additions, or change the order between the steps after understanding the spirit of the present disclosure.

In the present disclosure, features described and/or illustrated for one embodiment may be used in one or more other embodiments in a same way, or in combination with the features of other embodiments, or instead of the features of other embodiments.

The above descriptions are only preferred embodiments of the present disclosure and are not intended to limit the present disclosure, and various modifications and changes may be made by those skilled in the art. Any modification, equivalent replacement, improvement, and the like made within the spirit and principles of the present disclosure shall fall within the protection scope of the present disclosure.

## Claims

1. A device for acquiring and processing an electroencephalogram (EEG) signal, comprising:
an analog front-end processing circuit, comprising an analog front-end chip, wherein the analog front-end chip is configured to take a clock signal with a first clock frequency from an external clock as a clock input, acquire an EEG signal at a first sampling rate matching the first clock frequency to obtain an analog EEG signal, convert the analog EEG signal into a digital EEG signal, and transmit the digital EEG signal to a control unit, wherein a default sampling rate of the analog front-end chip is a second sampling rate different from the first sampling rate;
a frequency division circuit, connected to the external clock with the first clock frequency, and configured to perform N-fold frequency division on a clock crystal oscillator of the external clock to obtain a clock signal with a target clock frequency, wherein N is an even multiple of 2 and N=fl/fg, wherein f1 represents the first clock frequency, and fg represents the target clock frequency; and
the control unit configured to take the clock signal with the target clock frequency obtained after frequency division by the frequency division circuit as a clock input, and perform data processing on the digital EEG signal from the analog front-end chip.

2. The device of claim 1, further comprising:
a power supply circuit, configured to supply power for the analog front-end chip and the main control chip, wherein the power supply circuit comprises a main power supply module and an auxiliary power supply module,
wherein the main power supply module comprises an analog circuit power supply circuit and a digital circuit power supply circuit, wherein the analog circuit power supply circuit comprises a low dropout regulator, and is configured to provide a first voltage for an analog circuit of the EEG signal acquiring device to supply power; and the digital circuit power supply circuit comprises a buck circuit, and is configured to provide a first voltage for a digital circuit of the EEG signal acquiring device to supply power, and
the auxiliary circuit module is configured to divide a second voltage from the first voltage provided by the analog circuit power supply circuit to supply power for the analog front-end chip.

3. The device of claim 1, wherein the first clock frequency ranges from 1.5MHz to 2.25MHz, and the first sampling rate is F and satisfies: F = f1/2/k, wherein k is selected from 64, 128, 256, 512, 1024, 2048, or 4096.

4. The device of any one of claims 1 to 3, wherein the frequency division circuit comprises:
a first frequency division sub-circuit, connected to the external clock with the first clock frequency, and configured to perform M-fold frequency division on the clock crystal oscillator of the external clock, wherein M is an even multiple of 2; and
a second frequency division sub-circuit, configured to further perform L-fold frequency division on a signal after the M-fold frequency division by the first frequency division circuit to obtain the clock signal with the target clock frequency, wherein L is an even multiple of 2 and L=N/M.

5. The device of claim 3, wherein N is 64, and the frequency division circuit comprises:
a first frequency division sub-circuit, connected to the external clock with the first clock frequency, and configured to perform 4-fold frequency division on the clock crystal oscillator of the external clock;
a second frequency division sub-circuit, configured to further perform 4-fold frequency division on a signal after the 4-fold frequency division by the first frequency division circuit; and
a third frequency division sub-circuit, configured to further perform 4-fold frequency division on a signal after the 4-fold frequency division by the second frequency division circuit to obtain the clock signal with the target clock frequency.

6. The device of claim 1, wherein the analog front-end processing circuit further comprises:
an electrostatic discharge (ESD) protection circuit, disposed at an EEG signal input end for ESD protection; and
a low-pass filtering circuit, disposed at the EEG signal input end for filtering out high-frequency electromagnetic waves and high-frequency crosstalk.

7. The device of claim 2, wherein the analog front-end chip comprises a multi-channel EEG signal multiplexer (MUX) switching module configured to perform polling acquisition on the EEG signal;
the first voltage ranges from 1.8V to 3.6V;
the second voltage is represented as AVDD and satisfies: AVDD=(AVDD+)-(AVDD-), wherein AVDD+ and AVDD- represent a high voltage and a low voltage of the analog front-end chip respectively, and AVDD ranges from 4.75V to 5.25V.

8. A method for acquiring and processing an EEG signal, comprising:
taking, by an analog front-end chip, a clock signal with a first clock frequency from an external clock as a clock input, acquiring an EEG signal at a first sampling rate matching the first clock frequency to obtain an analog EEG signal, converting the analog EEG signal into a digital EEG signal, and transmitting the digital EEG signal to a control unit, wherein a default sampling rate of the analog front-end chip is a second sampling rate different from the first sampling rate;
connecting, by a frequency division circuit, the external clock with the first clock frequency, and performing N-fold frequency division on a clock crystal oscillator of the external clock to obtain a clock signal with a target clock frequency, wherein N is an even multiple of 2 and N=f1/fg, wherein f1 represents the first clock frequency, and fg represents the target clock frequency; and
taking, by the control unit, the clock signal with the target clock frequency obtained after frequency division by the frequency division circuit as a clock input, and performing data processing on the digital EEG signal from the analog front-end chip.

9. The method of claim 8, wherein the connecting, by a frequency division circuit, the external clock with the first clock frequency, and performing N-fold frequency division on a clock crystal oscillator of the external clock to obtain a clock signal with a target clock frequency comprises:
connecting, by the frequency division circuit, the external clock with the first clock frequency, and performing M-fold frequency division on the clock crystal oscillator of the external clock, wherein M is an even multiple of 2; and
performing L-fold frequency division on a signal after the M-fold frequency division by the first frequency division circuit to obtain the clock signal with the target clock frequency, wherein L is an even multiple of 2 and L=N/M.

10. The method of claim 8, wherein the first clock frequency ranges from 1.5MHz to 2.25MHz, N is 64, and the first sampling rate is F and satisfies: F = f1/2/k, wherein k is selected from 64, 128, 256, 512, 1024, 2048, or 4096;
wherein the connecting, by a frequency division circuit, the external clock with the first clock frequency, and performing N-fold frequency division on a clock crystal oscillator of the external clock to obtain a clock signal with a target clock frequency comprises:
connecting, by the frequency division circuit, the external clock of the first clock frequency, and performing a first 4-fold frequency division on the clock crystal oscillator of the external clock;
performing a second 4-fold frequency division on a signal after the first 4-fold frequency division; and
performing a third 4-fold frequency division on a signal after the second 4-fold frequency division to obtain the clock signal with the target clock frequency.
